# EUROPEAN PATENT SPECIFICATION

(11) **EP 1 004 572 B1**
(45) Date of publication and mention of the grant of the patent: **08.03.2006**
(21) Application number: 00200498.4
(22) Date of filing: 01.07.1994
(51) Int. Cl.: C07C 229/34, A61K 31/13, C07D 213/55, C07C 225/34

(54) **Amines as inhibitors of TNF alpha**
Aminen als Inhibitoren von TNF alpha
Amines en tant qu'inhibiteurs de TNF alpha

(30) Priority: 02.07.1993 US 87510
(43) Date of publication of application: 31.05.2000
(62) Divisional of application: 94921439.9
(73) Proprietor: CELGENE CORPORATION, Warren, New Jersey 07059 (US)
(72) Inventor: Muller, George W., Bridgewater, New Jersey (US)
(74) Representative: Jones Day

(56) References cited:
- CH-A- 554 833
- CH-A- 554 837
- US-A- 5 260 329
- HENRY V. SECOR ET AL.: "Nicotine analogues. synthesis of pyridylazetidines" JOURNAL OF ORGANIC CHEMISTRY., vol. 44, no. 18, 1979, pages 3136-3140, XP002202453 AMERICAN CHEMICAL SOCIETY. EASTON., US ISSN: 0022-3263
- CHEMICAL ABSTRACTS, vol. 70, no. 11, 17 March 1969 (1969-03-17) Columbus, Ohio, US; abstract no. 47380j, SCHUBERT, HANS W. ET AL.: "New syntheses of 2-amino-5,6-dihydro-4H-1,3-thiazines." XP002202455 & ARCH. PHARM. (WEINHEIM), vol. 301, no. 10, 1968, pages 750-762, -& DATABASE CAPLUS [Online] CHEMICAL ABSTRACTS SERVICE, COLUMBUS, OHIO, US; Database accession no. 70:47380j XP002202456
- ELISABETH SCHULZ ET AL.: "Synthese und einige pharmakologische Eigenschaften von Alkylestern verschiedener DL-omega-phenylamniosäuren" PHARMAZIE., vol. 38, no. 5, 1983, pages 310-313, XP002202454 VEB VERLAG VOLK UND GESUNDHEIT. BERLIN., DD ISSN: 0031-7144

## Description

### Background of the Invention

The present invention relates a method of reducing levels of TNF_{α} in a mammal and to compounds and compositions useful therein.

TNF_{α}, or tumor necrosis factor α, is a cytokine which is released primarily by mononuclear phagocytes in response to various immunostimulators. When administered to animals or humans it causes inflammation, fever, cardiovascular effects, hemorrhage, coagulation and acute phase responses similar to those seen during acute infections and shock states.

Excessive or unregulated TNF_{α} production has been implicated in a number of disease conditions. These include endo-toxemia and/or toxic shock syndrome {Tracey et al., Nature 330, 662-664 (1987) and Hinshaw et al., Circ. Shock 30,279-292 (1990)}; cachexia (Dezube et al., Lancet, 335 (8690), 662 (1990)}; and Adult Respiratory Distress Syndrome where TNF_{α} concentration in excess of 12,000 pg/mL have been detected in pulmonary aspirates from ARDS patients (Millar et al., Lancet 2(8665), 712-714 (1989)). Systemic infusion of recombinant TNF_{α} also resulted in changes typically seen in ARDS {Ferrai-Baliviera et al., Arch. Surg. 124 (12), 1400-1405 (1989)}.

TNF_{α} appears to be involved in bone resorption diseases, including arthritis where it has been determined that when activated, leukocytes will produce a bone-resorbing activity, and data suggest that TNF_{α} contributes to this activity. (Bertolini et al., Nature 319, 516-518 (1986) and Johnson et al., Endocrinology 124(3), 1424-1427 (1989).) It has been determined that TNF_{α} stimulates bone resorption and inhibits bone formation in vitro and in vivo through stimulation of osteoclast formation and activation combined with inhibition of osteoblast function. Although TNF_{α} may be involved in many bone resorption diseases, including arthritis, the most compelling link with disease is the association between production of TNF_{α} by tumor or host tissues and malignancy associated hypercalcemia {Calci. Tissue Int. (US) 46 (Suppl.), S3-10 (1990)}. In Graft versus Host Reaction, increased serum TNF_{α} levels have been associated with major complication following acute allogenic bone marrow transplants (Holler et al., Blood, 75(4), 1011-1016 (1990)).

Cerebral malaria is a lethal hyperacute neurological syndrome associated with high blood levels of TNF_{α} and the most severe complication occurring in malaria patients. Levels of serum TNF_{α} correlated directly with the severity of disease and the prognosis in patients with acute malaria attacks {Grau et al., N. Engl. J. Med. 320(24), 1586-1591 (1989)}.

TNF_{α} also plays a role in the area of chronic pulmonary inflammatory diseases. The deposition of silica particles leads to silicosis, a disease of progressive respiratory failure caused by a fibrotic reaction. Antibody to TNF_{α} completely blocked the silica-induced lung fibrosis in mice {Pignet et al., Nature, 344:245-247 (1990)}. High levels of TNF_{α} production (in the serum and in isolated macrophages) have been demonstrated in animal models of silica and asbestos induced fibrosis {Bissonnette et al., Inflammation 13(3), 329-339 (1989)}. Alveolar macrophages from pulmonary sarcoidosis patients have also been found to spontaneously release massive quantities of TNF_{α} as compared with macrophages from normal donors (Baughman et al., J. Lab. Clin. Med. 115(1), 36-42 (1990)).

TNF_{α} is also implicated in the inflammatory response which follows reperfusion, called reperfusion injury, and is a major cause of tissue damage after loss of blood flow (Vedder et al., PNAS 87, 2643-2646 (1990)). TNF_{α} also alters the properties of endothelial cells and has various pro-coagulant activities, such as producing an increase in tissue factor pro-coagulant activity and suppression of the anticoagulant protein C pathway as well as down-regulating the expression of thrombomodulin {Sherry et al., J. Cell Biol. 107, 1269-1277 (1988)}. TNF_{α} has pro-inflammatory activities which together with its early production (during the initial stage of an inflammatory event) make it a likely mediator of tissue injury in several important disorders including but not limited to, myocardial infarction, stroke and circulatory shock. Of specific importance may be TNF_{α}-induced expression of adhesion molecules, such as intercellular adhesion molecule (ICAM) or endothelial leukocyte adhesion molecule (ELAM) on endothelial cells (Munro et al., Am. J. Path. 135(1), 121-132 (1989)).

Moreover, it now is known that TNF_{α} is a potent activator of retrovirus replication including activation of HIV-1. {Duh et al., Proc. Nat. Acad. Sci. 86, 5974-5978 (1989); Poll et al., Proc. Nat. Acad. Sci. 87, 782-785 (1990); Monto et al., Blood 79, 2670 (1990); Clouse et al., J. Immunol. 142, 431-438 (1989); Poll et al., AIDS Res. Hum. Retrovirus, 191-197 (1992)}. AIDS results from the infection of T lymphocytes with Human Immunodeficiency Virus (HIV). At least three types or strains of HIV have been identified, i.e., HIV-1, HIV-2 and HIV-3. As a consequence of HIV infection, T-cell mediated immunity is impaired and infected individuals manifest severe opportunistic infections and/or unusual neoplasms. HIV entry into the T lymphocyte requires T lymphocyte activation. Other viruses, such as HIV-1, HIV-2 infect T lymphocytes after T cell activation and such virus protein expression and/or replication is mediated or maintained by such T cell activation. Once an activated T lymphocyte is infected with HIV, the T lymphocyte must continue to be maintained in an activated state to permit HIV gene expression and/or HIV replication. Cytokines, specifically TNF_{α}, are implicated in activated T-cell mediated HIV protein expression and/or virus replication by playing a role in maintaining T lymphocyte activation. Therefore, interference with cytokine activity such as by prevention or inhibition of cytokine production, notably TNF_{α}, in an HIV-infected individual aids in limiting the maintenance of T lymphocyte caused by HIV infection.

Monocytes, macrophages, and related cells, such as kupffer and glial cells, have also been implicated in maintenance of the HIV infection. These cells, like T cells, are targets for viral replication and the level of viral replication is dependent upon the activation state of the cells. (Rosenberg et al., The Immunopathogenesis of HIV Infection, Advances in Immunology, 57 (1989)). Cytokines, such as TNF_{α}, have been shown to activate HIV replication in monocytes and/or macrophages {Poli et al. Proc. Natl. Acad. Sci., 87, 782-784 (1990)}, therefore, prevention or inhibition of cytokine production or activity aids in limiting HIV progression as stated above for T cells. Additional studies have identified TNF_{α} as a common factor in the activation of HIV in vitro and has provided a clear mechanism of action via a nuclear regulatory protein found in the cytoplasm of cells (Osborn, et al., PNAS 86 2336-2340). This evidence suggests that a reduction of TNF_{α} synthesis may have an antiviral effect in HIV infections, by reducing the transcription and thus virus production.

AIDS viral replication of latent HIV in T cell and macrophage lines can be induced by TNF_{α} (Folks et al., PNAS 86, 2365-2368 (1989)). A molecular mechanism for the virus inducing activity is suggested by TNF_{α}'s ability to activate a gene regulatory protein (NFKB) found in the cytoplasm of cells, which promotes HIV replication through binding to a viral regulatory gene sequence (LTR) {Osborn et al., PNAS 86, 2336-2340 (1989)}. TNF_{α} in AIDS associated cachexia is suggested by elevated serum TNF_{α} and high levels of spontaneous TNF_{α} production in peripheral blood monocytes from patients (Wright et al. J. Immunol. 141(1), 99-104 (1988)}.

TNF_{α} has been implicated in various roles with other viral infections, such as the cytomegalia virus (CMV), influenza virus, adenovirus, and the herpes family of viruses for similar reasons as those noted.

Preventing or inhibiting the production or action of TNF_{α} is, therefore, predicted to be a potent therapeutic strategy for many inflammatory, infectious, immunological or malignant diseases. These include but are not restricted to septic shock, sepsis, endotoxic shock, hemodynamic shock and sepsis syndrome, post ischemic reperfusion injury, malaria, mycobacterial infection, meningitis, psoriasis, congestive heart failure, fibrotic disease, cachexia, graft rejection, cancer, autoimmune disease, opportunistic infections in AIDS, rheumatoid arthritis, rheumatoid spondylitis, osteoarthritis, other arthritic conditions, Crohn's disease, ulcerative colitis, multiple sclerosis, systemic lupus erythrematosis, ENL in leprosy, radiation damage, and hyperoxic alveolar injury. Efforts directed to the suppression of the effects of TNF_{α} have ranged from the utilization of steroids such as dexamethasone and prednisolone to the use of both polyclonal and monoclonal antibodies (Beutler et al., Science 234, 470-474 (1985); WO 92/11383).

The nuclear factor kB (NFKB) is a pleiotropic transcriptional activator (Lenardo, et al. Cell 1989, 58, 227-29). NFKB has been implicated as a transcriptional activator in a variety of disease and inflammatory states and is thought to regulate cytokine levels including but not limited to TNF_{α} and also to be an activator of HIV transcription (Dbaibo, et al. J. Biol. Chem. 1993, 17762-66; Duh et al. Proc. Natl. Acad. Sci. 1989, 86, 5974-78: Bachelerie et al. Nature 1991, 350, 709-12; Boswas et al. J.. Acquired Immune Deficiency Syndrome 1993, 6, 778-786; Suzuki et al. Biochem. And Biophys. Res. Comm. 1993, 193, 277-83; Suzuki et al. Biochem. And Biophys. Res Comm. 1992, 189, 1709-15; Suzuki et al. Biochem. Mol. Bio. Int. 1993, 31(4), 693-700; Shakhov et al. 1990, 171, 35-47; and Staal et al. Proc. Natl. Acad. Sci. USA 1990, 87, 9943-47). Thus, inhibition of NFKB binding can regulate transcription of cytokine gene(s) and through this modulation and other mechanisms be useful in the inhibition of a multitude of disease states. The compounds claimed in this patent can inhibit the action of NFKB in the nucleus and thus are useful in the treatment of a variety of diseases including but not limited to rheumatoid arthritis, rheumatoid spondylitis, osteoarthritis, other arthritic conditions, septic shock, septis, endotoxic shock, graft versus host disease, wasting, Crohn's disease, ulcerative colitis, multiple sclerosis, systemic lupus erythrematosis, ENL in leprosy, HIV, AIDS, and opportunistic infections in AIDS.

TNF_{α} and NFKB levels are influenced by a reciprocal feedback loop. As noted above, the compounds of the present invention affect the levels of both TNF_{α} and NFKB. It is not known at this time, however, how the compounds of the present invention regulate the levels of TNF_{α}, NFKB, or both.

### Detailed Description

The present invention is based on the discovery that a class of non-polypeptide imides more fully described herein appear to inhibit the action of TNF_{α}.

An aspect of the present invention pertains to the use of compounds of the formula: 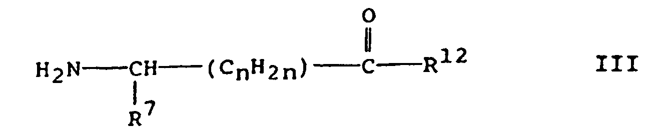 in the preparation of medicaments as claimed, wherein in the formula
R⁷ is (i) straight, branched, or cyclic alkyl of 1 to 12 carbon atoms, (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iii) benzyl unsubstituted or substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (iv) napthyl, (v) benzyloxy, or (vi) imidazol-4-ylmethyl;
R¹² is -OH, alkoxy of 1 to 12 carbon atoms, -O-CH₂-pyridyl, -O-benzyl, or 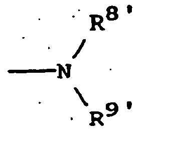
where n has a value of 0, 1, 2, or 3;
R^{8'} is hydrogen or alkyl of 1 to 10 carbon atoms; and
R^{9'} is hydrogen, alkyl of 1 to 10 carbon atoms, -CH₂₋pyridyl, benzyl, -COR¹⁰, or -SO₂R¹⁰ in which R¹⁰ is hydrogen, alkyl of 1 to 4 carbon atoms, or phenyl.

Another aspect of the present invention pertains to the compounds of the formula III described above per se and otherwise was claimed in the claims. The invention also pertains to the use of these compounds in therapy and in pharmaceutical composition containing these compounds as claimed.

Typical compounds of this invention include Ethyl 3-Amino-3- (3', 4' -dimethoxyphenyl) propionate; Methyl 3-amino-3-(3',4'-dimethoxyphenyl)propionate hydrochloride; Methyl (S)-3-amino-3-(3',4'-dimethoxyphenyl)propionate hydrochloride; Methyl (R) -3-amino-3-(3', 4' -dimethoxyphenyl) propionate hydrochloride;

The term alkyl as used herein denotes a univalent saturated branched or straight hydrocarbon chain. Unless otherwise stated, such chains can contain from 1 to 18 carbon atoms. Representative of such alkyl groups are methyl, ethyl, propyl, isopropyl, butyl, isobutyl, sec-butyl, tert-butyl, pentyl, isopentyl, neopentyl, tert-pentyl, hexyl, isohexyl, heptyl, octyl, nonyl, decyl, undecyl, dodecyl, tridecyl, tetradecyl, pentadecyl, hexadecyl, heptadecyl, octadecyl, and the like. When qualified by "lower" , the alkyl group will contain from 1 to 6 carbon atoms. The same carbon content applies to the parent term "alkane" and to derivative terms such as "alkoxy".

The compounds can be used, under the supervision of qualified professionals, to inhibit the undesirable effects of TNF_{α}. The compounds can be administered orally, rectally, or parenterally, alone or in combination with other therapeutic agents including antibiotics, steroids, etc., to a mammal in need of treatment. Oral dosage forms include tablets, capsules, dragees, and similar shaped, compressed pharmaceutical forms. Isotonic saline solutions containing 20-100 mg/mL can be used for parenteral administration which includes intramuscular, intrathecal, intravenous and intra-arterial routes of administration. Rectal administration can be effected through the use of suppositories formulated from conventional carriers such as cocoa butter.

Dosage regimens must be titrated to the particular indication, the age, weight, and general physical condition of the patient, and the response desired but generally doses will be from about 10 to about 500 mg/day as needed in single or multiple daily administration. In general, an initial treatment regimen can be copied from that known to be effective in interfering with TNF_{α} activity for other TNF_{α} mediated disease states by the compounds of the present invention. Treated individuals will be regularly checked for T cell numbers and T4/T8 ratios and/or measures of viremia such as levels of reverse transcriptase or viral proteins, and/or for progression of cytokine-mediated disease associated problems such as cachexia or muscle degeneration. If no effect is soon following the normal treatment regimen, then the amount of cytokine activity interfering agent administered is increased; e.g., by fifty percent a week.

The compounds of the present invention also can be used topically in the treatment or prophylaxis of topical disease states mediated or exacerbated by excessive TNF_{α} production, respectively, such as viral infections, such as those caused by the herpes viruses, or viral conjunctivitis, etc.

The compounds also can be used in the veterinary treatment of mammals other than humans in need of prevention or inhibition of TNF_{α} production. TNF_{α} mediated diseases for treatment, therapeutically or prophylactically, in animals include disease states such as those noted above, but in particular viral infections. Examples include feline immunodeficiency virus, equine infectious anaemia virus, caprine arthritis virus, visna virus, and maedi virus, as well as other lentiviruses.

Certain of these compounds possess centers of chirality and can exist as optical isomers. Both the racemates of these isomers and the individual isomers themselves, as well as diastereomers when there are two chiral centers, are within the scope of the present invention. The racemates can be used as such or can be separated into their individual isomers mechanically as by chromatography using a chiral absorbant. Alternatively, the individual isomers can be prepared in chiral form or separated chemically from a mixture by forming salts with a chiral acid, such as the individual enantiomers of 10-camphorsulfonic acid, camphoric acid, alpha-bromocamphoric acid, methoxyacetic acid, tartaric acid, diacetyltartaric acid, malic acid, pyrrolidone-5-carboxylic acid, and the like, and then freeing one or both of the resolved bases, optionally repeating the process, so as obtain either or both substantially free of the other; i.e., in a form having an optical purity of >95%.

The compounds can be prepared using methods which are known in general for the preparation of imides. However, the present invention also pertains to an improvement in the formation of the final compounds, as discussed below in greater detail.

An N-alkoxycarbonylimide and an amine thus are allowed to react in the presence of a base such as sodium carbonate or sodium bicarbonate substantially as described by Shealy et al., Chem. & Ind., (1965) 1030-1031) and Shealy et al., J. Pharm. Sci. 57, 757-764 (1968) to yield the N-substituted imide. Alternatively, a cyclic acid anhydride can be reacted with an appropriate amine to form an imide. Formation of a cyclic imide also can be accomplished by refluxing a solution of an appropriately substituted dicarboxylic acid monoamide in anhydrous tetrahydrofuran with N, N'-carbonyldiimidazole. In contrast to prior art methods which produced a yield of less than 50%, this reaction produces yields in excess of 60%, in some cases greater than 90%. This reaction also has broader applicability, being useful not only in the preparation of compounds of the present invention but also in the preparation of known compounds such as thalidomide.

Prevention or inhibition of production of TNF_{α} by these compounds can be conveniently assayed using anti-TNF_{α} antibodies. For example, plates (Nunc Immunoplates, Roskilde, DK) are treated with 5 *µ*g/mL of purified rabbit anti-TNF_{α} antibodies at 4°C for 12 to 14 hours. The plates then are blocked for 2 hours at 25°C with PBS/0.05% Tween containing 5 mg/mL BSA. After washing, 100 *µ*L of unknowns as well as controls are applied and the plates incubated at 4°C for 12 to 14. hours. The plates are washed and assayed with a conjugate of peroxidase (horseradish) and mouse anti-TNF_{α} monoclonal. antibodies, and the color developed with o-phenylenediamine in phosphate-citrate buffer containing 0.012% hydrogen peroxide and read at 492 nm.

The following examples will serve to further typify the nature of this invention but should hot be construed as a limitation in the scope thereof, which scope is defined solely by the appended claims.

### EXAMPLE 52

To 40 mL of stirred ethanol at 0°C under nitrogen was slowly added thionyl chloride (3.3 mL, 45 mmol) followed by addition of 3-amino-3-(3'-pyridyl)propionic acid (2.65 g, 15 mmol). The reaction mixture was allowed to slowly warm to room temperature and then refluxed for 3 hours. After 2 hours at reflux all of the solid had dissolved. The reaction mixture was allowed to cool to room temperature and stirred overnight. The slurry was filtered and the solid was washed with copious amounts of Ethanol. The solid was dried in vacuo (60°C, <1mm) to afford 3.17 g (79%) of Ethyl 3-amino-3-(3'-pyridyl)-propionate hydrochloride as a white powder: 1H NMR (DMSO-d₆, 250 MHz) δ 9.32 .(br s, 3 H), 9.21 (br s, 1 H), 8.87-8.95 (m, 2 H), 8.09-8.14 (m, 1 H), 4.93 (br s, 1 H), 3.90-4.15 (m, 2 H), 3.20-3.38 (m, 2 H), 1.11 (t, 3 H, J = 7 Hz); 13C NMR (DMSO-d₆) δ 168.8, 144.5, 142.8, 142.6, 136.2, 126.7, 60.7, 47.9, 37.2, 13.9.

### EXAMPLE 56

The procedure of Example 52 was followed except the reaction was run at room temperature. Ethyl 3-Amiho-3-(3',4'-dimethoxyphenyl)propionate was isolated as a white powder (2.04 g, 88%): 1H NMR (DMSO-d₆, 250 MHz) δ 8.75 (br s', 3 H), 7.30-7.35 (m, 1 H), 6.90-7.05 (m, 2 H), 4.50 (dd, 1 H, J1 = 6 Hz, J2 = 9 Hz), 3.90-4.10 (m, 2 H), 3.77 (s, 3 H), 3.75 (s, 3 H), 3.19 (dd, 1 H, J1 = 6 Hz, J2 = 16 Hz), 2.98 (dd, 1 H, J1 = 9 Hz, J2 = 16 Hz), 1.10 (t, 3 H, J = 7 Hz); 13C NMR (DMSO-d₆) δ 169.1, 149.0, 148.6, 128.9, 120.1, 111.4, 60.4, 55.6, 55.5, 50.9, 38.7, 13.9.

### EXAMPLE 84

To 150 mL of stirred methanol at 0 °C under nitrogen was slowly added thionyl chloride (14.2 mL, 194.4 mmol). To the reaction mixture was then added 3-amino-3-(3',4'-dimethoxyphenyl)propionic acid (15.5 g, 64.8 mmol). The reaction mixture was stirred at 0 °C for 30 minutes and then allowed to warm to room temperature, and stirred overnight. The reaction solution was concentrated to an oil and then diluted with 200 mL of CH₃OH/Et₂O (1/3) and stirred. The resulting slurry was filtered and the solid was washed with a copious amount of ether. The solid was dried in vacuo (60 °C, < 1 mm) to afford 1.83 g (66%) of Methyl 3-amino-3-(3',4'-dimethoxyphenyl)propionate hydrochloride as a white powder: ¹ H NMR (dmso-d₆, 250 MHz) δ 8.59 (br s, 3 H, NH₃), 6.9-7.3 (m, 3 H, Ar), 4.52 (overlapping dd, 1 H), 3.77 (s, 3 H, OCH₃), 3.75 (s, 3 H, OCH₃), 3.57 (s, 3 H, OCH₃), 3.16 (dd, 1 H, J₁ = 6 Hz, J₂ = 16 Hz), 2.98 (dd, 1 H, J₁ = 8 Hz, J₂ = 16 Hz); ¹³C NMR (dmso-d₆) δ 169.6, 149.0, 129.0, 120.0, 111.5, 111.4, 55.7, 55.5, 51.7, 50.8, 38.5. Anal. Calcd for C₁₂H₁₈NO₄Cl. Theoretical C, 52.27; H, 6.58; N, 5.08. Found C, 52.44; H, 6.53; N, 5.01.

### EXAMPLE 86

To a stirred solution of benzaldehyde (1.58 mL, 15.5 mmol) in 10 mL of absolute ethanol at room temperature under nitrogen was added (R)-a-methylbenzylamine (2.0 mL, 15.51 mmol, 99% ee.). The reaction mixture was stirred for 3 hours. The reaction solution was then dried over magnesium sulfate and diluted to a 60 mL volume with EtOH. Ethanol washed Raney-Ni (⁻ 1.5 g) was added and the resulting suspension was treated with 58 psi of H₂ in a Parr Type Shaker. After 1 day, additional Raney-Ni (- 1 g) and 30 mL of ethanol were added and the hydrogenolysis continued for 3 days. The reaction mixture was filtered through Celite to remove the catalyst and concentrated to afford 3.11 g (95%) of N-Benzyl (R)-a-methylbenzylamine as a pale yellow oil contaminated with 5% of benzyl alcohol and (R)-a-methylbenzylamine; ¹H NMR (dmso-d₆, 250 MHz) δ 7.1-7.5 (m, 10 H, Ar), 3.68 (q, 1 H, J = 6.6 Hz), 3.48 (dd, 2 H, J₁ = 13.6 Hz, J₂ = 20.5 Hz), 1.26 (d, 3 H, J = 6.6 Hz, CH₃); ¹³C NMR (dmso-d₆) δ 146.1, 141.0, 128.2, 128.0, 127.8, 126.5, 126.4, 56.7, 50.6, 24.5. This mixture was used directly in the next reaction.

### EXAMPLE 87

To stirred solution of N-benzyl (R) -a-methylbenzylamine (1.9 g, 9.0 mmol) in 50 mL of tetrahydrofuran at 0 °C under nitrogen was added n-butyl lithium (1.6 M in hexanes; 9.0 mmol). The resulting red solution was stirred at O °C for 15 min and then cooled to -78 °C. To the reaction mixture was then dropwise added methyl trans-3-(3',4'-dimethoxyphenyl) - propion-2-enate (1.33 g, 6.0 mmol) in 20 mL tetrahydrofuran and the mixture was stirred for 15 in at -78 °C to afford a yellow solution. The reaction was then quenched by the addition of saturated ammonium chloride (20 mL). The mixture was allowed to warm to room temperature and poured into 40 mL of saturated sodium chloride (aq). The mixture was extracted with ether (2x 60 mL) and the combined organic layers dried (MgSO₄) and concentrated to afford 3.35 g of crude product as a yellow oil. The oil was purified by flash chromatography (silica gel, hexane/CH₂Cl₂, 30-0%, (v/v)) to afford 1.24 g (48%) of Methyl (S)-N-Benzyl-N-(R)-a-methylbenzyl-3-(3',4'-dimethoxyphenyl) propionate adduct as colorless oil: ¹H NMR (dmso-d₆, 250 MHz) δ 6.7-7.5 (m, 13 H, Ar), 3.9-4.2 (m, 2 H), 3.78 (s, 3 H, OCH₃), 3.73 (s, 3 H, OCH₃), 3.65 (s, 2 H), 3.43 (s, 3 H, OCH₃), 2.6-2.9 (m, 2 H), 1.03 (d, 3 H, J = 7 Hz); ¹³C NMR (dmso-d₆) δ 171.6, 148.3, 147.8, 144.6, 141.5, 133.6, 128.1, 128.0, 127.7, 127.5, 126.7, 126.4, 119.6, 111.9, 111.2, 58.2, 56.4, 55.4, 55.3, 51.1, 49.7, 35.6, 17.1.

### EXAMPLE 88

Debenzylation of the above pure adduct was done following the literature procedure S. G. Davies and O. Ichihara (Tetrahedron Asymmetry 1991, 2, 183.). To a stirred solution methyl (S) - 3- (N-benzyl-N-(R)-a-methylbenzylamino)-3-(3',4'-dimethoxyphenyl)propionate (1.20 g, 2.77 mmol) in a mixture of MeOH (20 mL), water (2 mL) and acetic acid (0.5 mL) was added 20% palladium hydroxide on charcoal. The reaction mixture was treated with hydrogen (54 psi) at room temperature for 23 h on a Parr Type Shaker. The reaction mixture was filtered through Celite and then concentrated to afford the product as an acetate salt. The salt was dissolved in 10 mL of water, stirred with 0.7 mL 4 N HCl and then concentrated to a white solid. The solid was diluted with 40 mL of ether and stirred for 20 min. The slurry was filtered and the solid was dried in vacuo (room temperature, < 1 mm) to afford 0.57 g (75%) of Methyl (S)-3-amino-3- (3', 4' -dimethoxyphenyl) propionate hydrochloride as a white solid: HPLC 96% ee (Chiral Crownpack CR+ column); ¹H NMR (dmso-d₆, 250 MHz) δ 8.73 (br s, 3 H, NH₃), 6.90-7.40 (m, 3 H, Ar)., 4.51 (dd, 1 H, J₁ = 6 Hz, J₂ = 8 Hz), 3.77 (s, 3 H, OCH₃), 3.75 (s, 3 H, OCH₃), 3.56 (s, 3 H, OCH₃), 3.2 (dd, 1 H, J₁ = 6 Hz, J₂ = 16 Hz), 3.0 (dd, 1 H, J₁= 8 Hz, J₂ = 16 Hz); ¹³C NMR (dmso-d₆) δ 169.6, 149.0, 148.7, 129.0, 120.0, 111.5, 111.4, 55.7, 55.5, 51,7, 50.8, 38.6. Anal. Calcd for C₁₂H₁₈NO₄Cl-0.48 H₂O. Theoretical C, 50.67; H, 6.72; N, 4.92. Found C, 50.67; H, 6.46; N, 4.83.

### EXAMPLE 90

Prepared as described earlier for N-benzyl-(R)-a-methylbenzylamine from benzaldehyde (3.94 mL, 38.8 mmol) and (S)-a-methylbenzylamine (5.0 mL, 38.8 mmol, 96% ee.) to afford 7.88 g (96%) of N-Benzyl-(S)-a-methylbenzylamine as an oil contaminated with 5% of benzyl alcohol and (S)-a-methylbenzylamine: ¹H NMR (dmso-d₆, 250 MHz) δ 7.15-7.45 (m, 10 H, Ar), 3.69 (q, 1 H, J = 6.5 Hz), 3.48 (dd, 2 H, J₁ = 13.6 Hz, J₂ = 20.9 Hz), 2.45 (br s, 1 H, NH), 1.26 (d, 3 H, J = 6.5 Hz, CH₃); ¹³C NMR (dmso-d₆) δ 146.0, 141.0, 128.1, 128.0, 127.8, 126.4, 126.3, 56.7, 50.6, 24.5. This mixture was directly used in the next reaction.

### EXAMPLE 91

Prepared as described earlier as methyl (S)-3-(N-benzyl-N-(R)-a-methylbenzyl)-3-(3',4'-dimethoxyphenyl)propionate from butyl lithium (1.6 M in hexanes; 8.44 mmol), N-benzyl-(S)-a-methylbenylamine (1.78 g, 8.44 mmol) and 3-(3', 4'-dimethoxyphenyl)propyl-2-enate (1.50 g, 6.75 mmol) to afford 3.7 g of the crude product as a yellow oil. The oil was purified by flash chromatography (silica gel, ether/hexane, 20/80) to afford 0.57 g (20%) of Methyl (R)-3-(N-benzyl-N-(S)-a-methylbenzylamino)-3- (3', 4'-dimethoxyphenyl) propionate as a colorless oily ¹H NMR (dmgo-d₆, 250 MHz) δ 6.80-7.50 (m, 13 H, Ar), 4.15 (dd, 1 H, J₁ =6 Hz, J₂ = 9 Hz), 4.04 (q, 1 H, J = 7 Hz), 3.78 (s, 3 H, OCH₃), 3.73 (s, 3 H, OCH₃) 3.69 (s, 2 H), 3.43 (s, 3 H, OCH₃), 2.87 (dd, 1 H, J₁ =6 Hz, J₂ = 15 Hz), 2.67 (dd, 1 H, J₁ = 9 Hz, J₂ = 15 Hz), 1.04 (d, 3 H, J = 7 Hz, CH₃); ¹³C NMR (dmso-d₆) δ 171.6, 148.4, 147.8, 144.7, 141.5, 133.6, 128.1, 128.0, 127.8, 127.5, 126.7, 126.4, 119.6, 111.9, 111.2, 58.2, 56.4, 55.4, 55.3, 51.1, 49.7, 35.6, 17.1.

### EXAMPLE 92

Debenzylation of the above adduct was done following the literature procedure of S. G. Davies and O. Ichihara (Tetrahedron Asymmetry 1991, 2, 183.). A solution of methyl (R)-3-(N-benzyl-N-(S)-a-methylbenzylamino)-3-(3',4'-dimethoxyphenyl) propionate (0.57 g, 1.3 mmol) in MeOH (10. mL), water (1 mL) and acetic acid (0.25 mL) in the presence of 20% palladium hydroxide on charcoal was treated with hydrogen (59 psi) at room temperature for 23 h in a Parr Type Shaker. The mixture was filtered through Celite and then concentrated to afford the primary amine in an acetate salt, which was dissolved in 10 mL of water, stirred with 0.32 mL (4 N) HCl and concentrated to a white solid. To the solid was added 10 mL of ether and the mixture stirred for 30 min. The slurry was filtered, the solid was dried in vacuo (room temperature, 1 mm) to afford 0.32 g (90%) of Methyl (R)-3-amino-3-(-3',4'-dimethoxyphenyl) propionate hydrochloride as a white powder: Chiral HPLC (Crownpak Cr+ Column 92% ee; ¹H NMR (dmso-d₆, 250 MHz) δ 8.61 (br s, 3 H, NH₃), 6.90-7.30 (m, 3 H, Ar), 4.53 (br s, 1 H), 3.77 (s, 3 H, OCH₃), 3.75 (s, 3 H, OCH₃), 3.57 (s, 3 H, OCH₃), 3.16 (dd, 1 H, J₁ = 6 Hz, J₂ = 16 Hz), 2.98 (dd, 1 H, J₁ = 8 Hz, J₂ = 16 Hz); ¹³C NMR, (dmso-d₆) δ 169.5, 149.0, 148.6, 128.9, 119.8, 111.4, 111.2, 55.6, 55.4, 51.7, 50.7, 38.4. Anal.Calcd for C₁₂H₁₈NO₄Cl @0.48 H₂O. Theoretical C, 50.67; H, 6.72; N, 4.92. Found C, 50.66; H, 6.54; N, 4.81.

### EXAMPLE 94

Tablets, each containing 50 mg of active amine ingredient, can be prepared in the following manner:

| Constituents (for 1000 tablets) | |
|---|---|
| active amine ingredient | 50.0 g |
| lactose | 50.7 g |
| wheat starch | 7.5 g |
| polyethylene glycol 6000 | 5.0 g |
| talc | 5.0 g |
| magnesium stearate | 1.8 g |
| demineralized water | q.s. |

The solid ingredients are first forced through a sieve of 0.6 mm mesh width. The active amine ingredient, the lactose, the talc, the magnesium stearate and half of the starch then are mixed. The other half of the starch is suspended in 40 mL of water and this suspension is added to a boiling solution of the polyethylene glycol in 100 mL of water. The resulting paste is added to the pulverulent substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 95

Tablets, each containing 100 mg of active amine ingredient, can be prepared in the following manner:

| Constituents (for 1000 tablets) | |
|---|---|
| active amine ingredient | 100.0 g |
| lactose | 100.0 g |
| wheat starch | 47.0 g |
| magnesium stearate | 3.0 g |

All the solid ingredients are first forced through a sieve of 0.6 mm mesh width. The active amine ingredient, the lactose, the magnesium stearate and half of the starch then are mixed. The other half of the starch is suspended in 40 mL of water and this suspension is added to 100 mL of boiling water. The resulting paste is added to the pulverulent substances and the mixture is granulated, if necessary with the addition of water. The granulate is dried overnight at 35°C, forced through a sieve of 1.2 mm mesh width and compressed to form tablets of approximately 6 mm diameter which are concave on both sides.

### EXAMPLE 96

Tablets for chewing, each containing 75 mg of active amine ingredient, can be prepared in the following manner:

| Composition (for 1000 tablets) | |
|---|---|
| active amine ingredient | 75.0 g |
| mannitol | 230.0 g |
| lactose | 150.0 g |
| talc | 21.0 g |
| glycine | 12.5 g |
| stearic acid | 10.0 g |
| saccharin | 1.5 g |
| 5% gelatin solution | q.s. |

All the solid ingredients are first forced through a sieve of 0.25 mm mesh width. The mannitol and the lactose are mixed, granulated with the addition of gelatin solution, forced through a sieve of 2 mm mesh width, dried at 50°C and again forced through a sieve of 1.7 mm mesh width. The active amine ingredient, the glycine and the saccharin are carefully mixed, the mannitol, the lactose granulate, the stearic acid and the talc are added and the whole is mixed thoroughly and compressed to form tablets of approximately 10 mm diameter which are concave on both sides and have a breaking groove on the upper side.

### EXAMPLE. 97

Tablets, each containing 10 mg of active amine ingredient, can be prepared in the following manner:

| Composition (for 1000 tablets) | |
|---|---|
| active amine ingredient | 10.0 g |
| lactose | 328.5 g |
| corn starch | 17.5 g |
| polyethylene glycol 6000 | 5.0 g |
| talc | 25.0 g |
| magnesium stearate | 4.0 g |
| demineralized water | q.s. |

The solid ingredients are first forced through a sieve of 0.6 mm mesh width. Then the active amine ingredient, lactose, talc, magnesium stearate and half of the starch are intimately mixed. The other half of the starch is suspended in 65 mL of water and this suspension is added to a boiling solution of the polyethylene glycol in 260 mL of water. The resulting paste is added to the pulverulent substances, and the whole is mixed and granulated, if necessary with the addition of water. The granulate is dried overnight at 35°C, forced through a sieve of 1.2 mm mesh width and compressed to form tablets of approximately 10 mm diameter which are concave on both sides and have a breaking notch on the upper side.

### EXAMPLE 98

Gelatin dry-filled capsules, each containing 100 mg of active amine ingredient, can be prepared in the following manner:

| Composition (for 1000 capsules) | |
|---|---|
| active amine ingredient | 100.0 g |
| microcrystalline cellulose | 30.0 g |
| sodium lauryl sulphate | 2.0 g |
| magnesium stearate | 8.0 g |

The sodium lauryl sulphate is sieved into the active amine ingredient through a sieve of 0.2 mm mesh width and the two components are intimately mixed for 10 minutes. The microcrystalline cellulose is then added through a sieve of 0.9 mm mesh width and the whole is again intimately mixed for 10 minutes. Finally, the magnesium stearate is added through a sieve of 0.8_min width and, after mixing for a further 3 minutes, the mixture is introduced in portions of 140 mg each into size 0 (elongated) gelatin dry-fill capsules.

### EXAMPLE 99

A 0.2% injection or infusion solution can be prepared, for example, in the following manner:

| | |
|---|---|
| active amine ingredient | 5.0 g |
| sodium chloride | 22.5 g |
| phosphate buffer pH 7.4 | 300.0 g |
| demineralized water | to 2500.0 mL |

The active amine ingredient is dissolved in 1000 mL of water and filtered through a microfilter. The buffer solution is added and the whole is made up to 2500 mL with water. To prepare dosage unit forms, portions of 1.0 or 2.5 mL each are introduced into glass ampoules (each containing respectively 2.6 or 5.0 mg of imide).

## Claims

1. A compound of the formula: 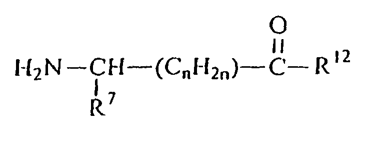
in which
R⁷ is (i) cyclic alkyl of 1 to 12 carbon atoms; (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of I to 10 carbon atoms, or halo; (iii) benzyl or benzyl substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of I to 4 carbon atoms, or halo; (iv) naphthyl; (v) benzyloxy; or (vi) imidazol-4-ylmethyl;
R¹² is alkoxy of 1 to 12 carbon atoms, -O-CH₂-pyridyl, -O- benzyl, or 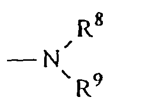 R⁸ is hydrogen or alkyl of 1 to 10 carbon atoms.
R⁹ is hydrogen, alkyl of 1 to 10 carbon atoms, -CH₂-pyridyl, benzyl, -COR¹⁰, or -SO₂R¹⁰, in which R¹⁰ is hydrogen, alkyl of 1 to 4 carbon atoms, or phenyl;
n has a value of 0, 1 , 2 or 3,
provided that the compound is not:
a) 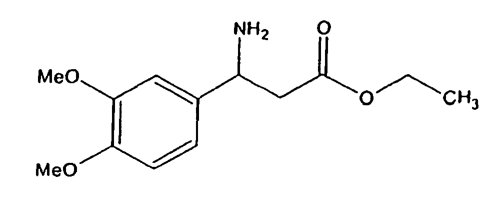
b) 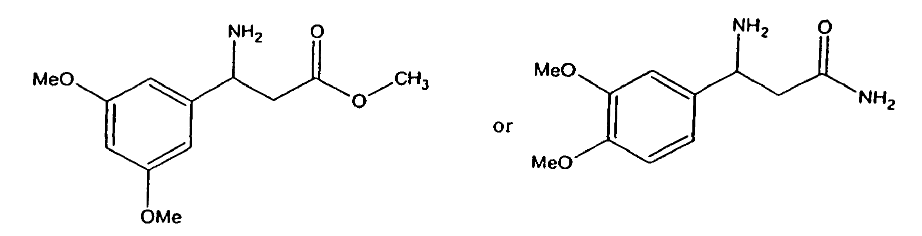
c) 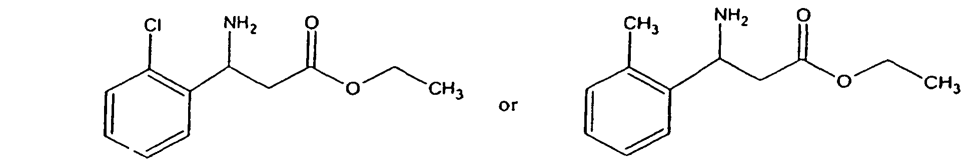 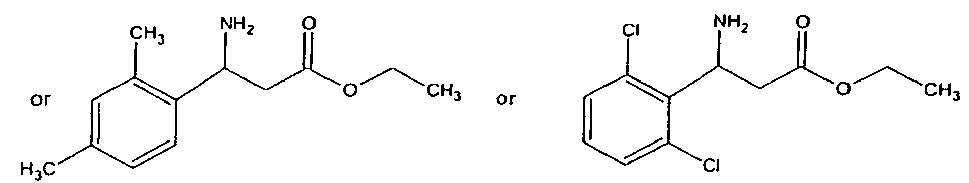 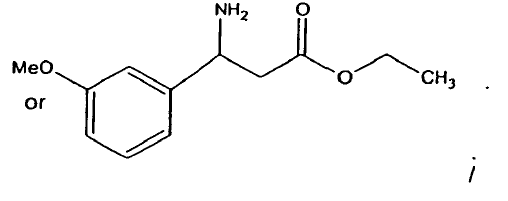 and provided that the compound is not a compound wherein R⁷ is benzyl and n is simultaneously : O and R¹² is simultaneously octyl.

2. A compound according to claim 1 for use in therapy.

3. Use of a compound of the formula: 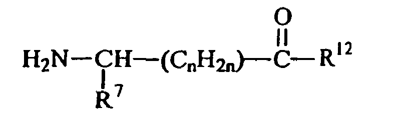
in which
R⁷ is (i) cyclic alkyl of I to 12 carbon atoms; (ii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo; (iii) benzyl or benzyl substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo; (iv) naphthyl; (v) benzyloxy; or (vi) imidazol-4-ylmethyl;
R¹² is alkoxy of 1 to 12 carbon atoms, -O-CH₂-pyridyl, -O- benzyl, or 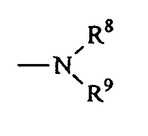
R⁸ is hydrogen or alkyl of 1 to 10 carbon atoms;
R⁹ is hydrogen, alkyl of 1 to 10 carbon atoms, -CH₂-pyridyl, benzyl, -COR¹⁰, or -SO₂R¹⁰, in which R¹⁰ is hydrogen, alkyl of 1 to 4 carbon atoms, or phenyl;
n has a value of 0, 1, 2 or 3;
in the manufacture of a medicament for reducing levels of TNFα in a mammal.

4. Use of a compound as defined in claim 3 in the manufacture of a medicament for inhibiting TTIFα-activated retrovirus replication in mammals.

5. A pharmaceutical composition comprising an amount of a compound according to claim 1 effective upon single or multiple dosage to inhibit TNFα.

## Patentansprüche

1. Eine Verbindung der Formel: 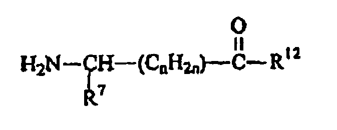 worin
R⁷ bedeutet: (i) ein zyklisches Alkyl mit 1 bis 12 Kohlenstoffatomen; (ii) ein mit ein oder mehr Substituenten substituiertes Phenyl, wobei jeder der Substituenten unabhängig von den anderen ausgewählt ist aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen; (iii) Benzyl oder Benzyl substituiert mit ein bis drei Substituenten ausgewählt aus der Gruppe bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen; (iv) Naphthyl, (v) Benzyloxy; oder (vi) Imidazol-4-ylmethyl;
R¹² Alkoxy mit 1 bis 12 Kohlenstoffatomen, -O-CH₂-Pyridyl, -O-Benzyl, oder 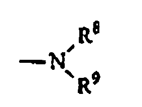 bedeutet;
R⁸ Wasserstoff oder Alkyl mit 1 bis 10 Kohlenstoffatomen bedeutet;
R⁹ Wasserstoff, Alkyl mit 1 bis 10 Kohlenstoffatomen, -CH₂-Pyridyl, Benzyl, -COR¹⁰, oder -SO₂R¹⁰ bedeutet, wobei R¹⁰ Wasserstoff, Alkyl mit 1 bis 4 Kohlenstoffatomen oder Phenyl bedeutet;
n einen Wert von 0, 1, 2 oder 3 hat,
mit der Bedingung, dass die Verbindung nicht eine der folgenden ist:
a) 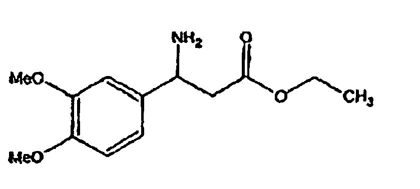
b) 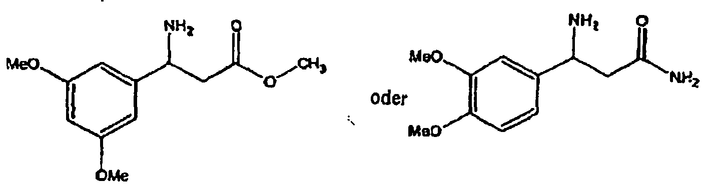
c) 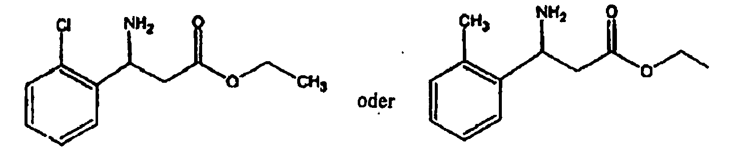 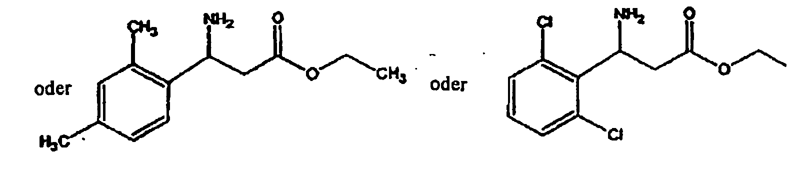 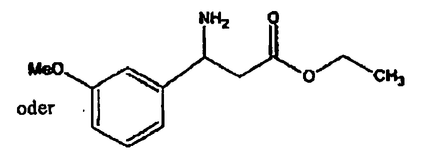 und mit der Bedingung, dass die Verbindung nicht eine Verbindung ist, in der R⁷ ein Benzyl ist und n gleichzeitig = 0 und R¹² gleichzeitig ein Octyl ist.

2. Eine Verbindung gemäß Anspruch 1 zur therapeutischen Verwendung.

3. Verwendung einer Verbindung der Formel: 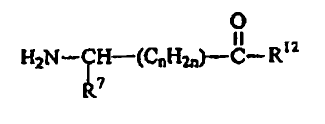 in der Herstellung eines Medikaments zur Reduzierung der TNFα-Konzentration in einem Säutetier, worin in der Formel
R⁷ bedeutet: (i) ein zyklisches Alkyl mit 1 bis 12 Kohlenstoffatomen; (ii) ein mit ein oder mehr Substituenten substituiertes Phenyl, wobei jeder der Substituenten unabhängig von den anderen ausgewählt ist aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 10 Kohlenstoffatomen, Alkoxy mit 1 bis 10 Kohlenstoffatomen oder Halogen; (iii) Benzyl oder Benzyl substituiert mit ein bis drei Substituenten ausgewählt aus der Gruppe bestehend aus Nitro, Cyano, Trifluormethyl, Carbethoxy, Carbomethoxy, Carbopropoxy, Acetyl, Carbamoyl, Acetoxy, Carboxy, Hydroxy, Amino, Alkyl mit 1 bis 4 Kohlenstoffatomen, Alkoxy mit 1 bis 4 Kohlenstoffatomen oder Halogen; (iv) Naphthyl, (v) Benzyloxy; oder (vi) Imidazol-4-ylmethyl;
R¹² Alkoxy mit 1 bis 12 Kohlenstoffatomen, -O-CH₂-Pyridyl, -O-Benzyl, oder 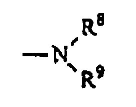 bedeutet;
R⁸ Wasserstoff oder Alkyl mit 1 bis 10 Kohlenstoffatomen bedeutet;
R⁹ Wasserstoff, Alkyl mit 1 bis 10 Kohlenstoffatomen, -CH₂-Pyridyl, Benzyl, -COR¹⁰, oder -SO₂R¹⁰ bedeutet, wobei R¹⁰ Wasserstoff, Alkyl mit 1 bis 4 Kohlenstoffatomen oder Phenyl bedeutet;
n einen Wert von 0, 1, 2 oder 3 hat.

4. Verwendung einer Verbindung, wie sie in Anspruch 3 definiert ist, in der Herstellung eines Medikaments zur Inhibierung von TNFα-aktivierter Retrovirus-Replikation in Säugetieren.

5. Eine pharmazeutische Zusammensetzung umfassend eine wirksame Menge einer Verbindung gemäß Anspruch 1 zur Inhibierung von TNFα in einer Einzel- oder Vielfach-Dosiereinheit.

## Revendications

1. Composé de formule : 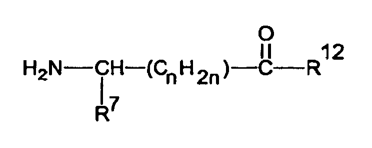 dans lequel
R⁷ est (i) un alkyle cyclique de 1 à 12 atomes de carbone ; (ii) un phényle substitué par un ou plusieurs substituants choisis chacun indépendamment de l'autre parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone, ou halogéno ; (iii) un benzyle ou un benzyle substitué par un à trois substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone, ou halogéno ; (iv) un naphtyle ; (v) un benzyloxy ; ou (vi) un imidazol-4-ylméthyle ;
R¹² est un alcoxy de 1 à 12 atomes de carbone, -O-CH₂-pyridyle, -O-benzyle, ou 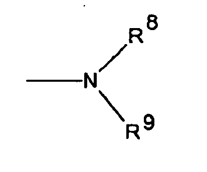
R⁸ est l'hydrogène ou un alkyle de 1 à 10 atomes de carbone ;
R⁹ est l'hydrogène, un alkyle de 1 à 10 atomes de carbone, -CH₂-pyridyle, un benzyle, -COR¹⁰, ou -SO₂R¹⁰,
dans lequel R¹⁰ est l'hydrogène, un alkyle de 1 à 4 atomes de carbone, ou un phényle ;
n vaut 0, 1, 2 ou 3,
pourvu que le composé ne soit pas :
a) 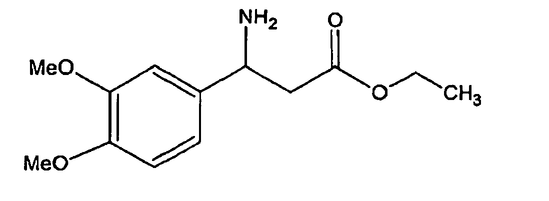
b) 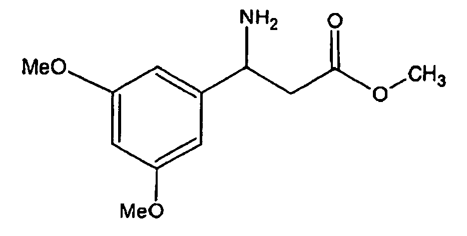 ou 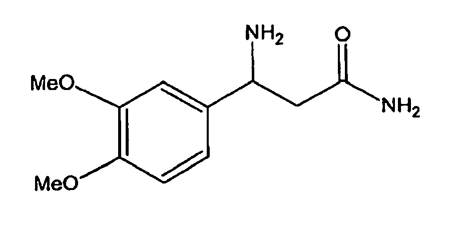
c) 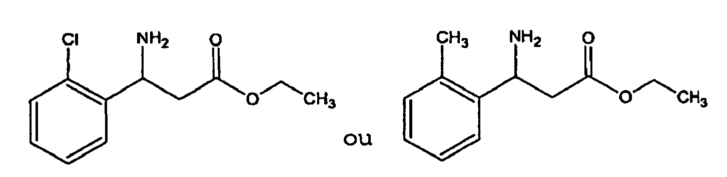 ou 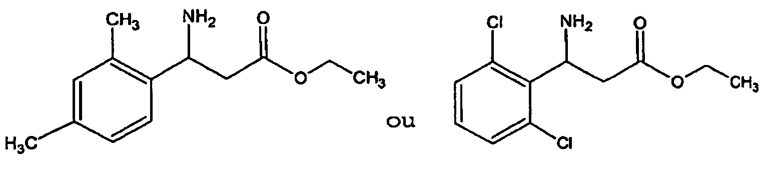 ou 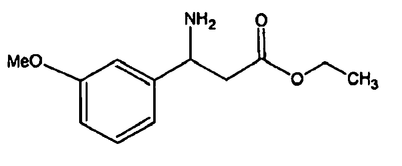 et pourvu que le composé ne soit pas un composé dans lequel R⁷ est un benzyle et n'est simultanément égal à 0 et R¹² est simultanément un octyle.

2. Composé selon la revendication 1 destiné à être utilisé en traitement.

3. Utilisation d'un composé de formule : 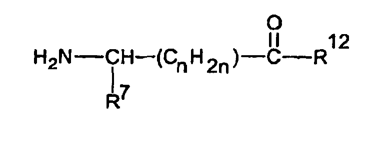 dans lequel
R⁷ est (i) un alkyle cyclique de 1 à 12 atomes de carbone ; (ii) un phényle substitué par un ou plusieurs substituants choisis chacun indépendamment de l'autre parmi les groupes nitro, cyano, trifluorométhyle, carbéthoxy, carbométhoxy, carbopropoxy, acétyle, carbamoyle, acétoxy, carboxy, hydroxy, amino, alkyle de 1 à 10 atomes de carbone, alcoxy de 1 à 10 atomes de carbone, ou halogéno ; (iii) un benzyle ou un benzyle substitué par un à trois substituants choisis dans le groupe constitué par les groupes nitro, cyano, trifluorométhyle, carbéthoxy, carbométhoxy, carbopropoxy, acétyle, carbamoyle, acétoxy, carboxy, hydroxy, amino, alkyle de 1 à 4 atomes de carbone, alcoxy de 1 à 4 atomes de carbone, ou halogéno ; (iv) un naphtyle ; (v) un benzyloxy ; ou (vi) un imidazol-4-ylméthyl ;
R¹² est un alcoxy de 1 à 12 atomes de carbone, -O-CH₂-pyridyle, -O-benzyle, ou 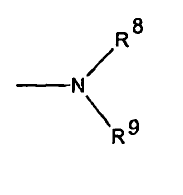
R⁸ est l'hydrogène ou un alkyle de 1 à 10 atomes de carbone ;
R⁹ est l'hydrogène, un alkyle de 1 à 10 atomes de carbone, -CH₂-pyridyle, un benzyle, -COR¹⁰, ou -SO₂R¹⁰,
dans lequel R¹⁰ est l'hydrogène, un alkyle de 1 à 4 atomes de carbone, ou un phényle ;
n vaut 0, 1, 2 ou 3 ;
dans la fabrication d'un médicament permettant de réduire les niveaux de TNF_{α} chez un mammifère.

4. Utilisation d'un composé tel que défini dans la revendication 3 dans la fabrication d'un médicament permettant d'inhiber la réplication des rétrovirus activée par le TNFα chez les mammifères.

5. Composition pharmaceutique comprenant une quantité d'un composé selon la revendication 1 efficace en dose unique ou multiple pour inhiber le TNFα.
